# DEMANDE DE BREVET EUROPEEN

(11) **EP 2 601 942 A1**
(43) Date de publication de la demande: **12.06.2013**
(21) Numéro de dépôt: 12195997.7
(22) Date de dépôt: 07.12.2012
(51) Int. Cl.: A61K 31/13, A61K 31/403, A61P 25/28

(54) **Nouvelle association entre le 4-{3-[CIS-hexahydrocyclopenta[C]pyrrol-2(1H)-yl]propoxy}benzamide et un antagoniste des récepteurs NMDA et les compositions pharmaceutiques qui la contiennent**

(30) Priorité: 09.12.2011 FR 1103777; 09.12.2011 US 201161568831 P
(71) Demandeur: Les Laboratoires Servier, 92284 Suresnes Cedex (FR)
(72) Inventeur: Sors, Aurore, 75015 Paris (FR); Trocme-Thibierge, Caryn, 92270 Bois Colombes (FR); Merdes, Annette, 81377 Munich (DE)

(57) **Abrégé**

Association entre le 4-{3-[*cis*-hexahydrocyclopenta[*c*]pyrrol-2(1*H*)-yl]propoxy}benzamide de formule (I): 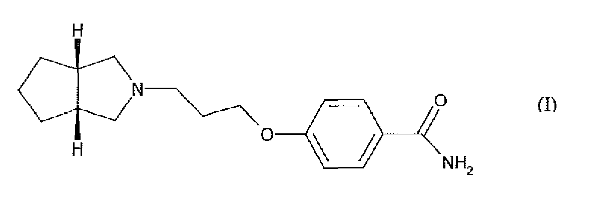 ou un de ses sels d'addition à un acide ou à une base pharmaceutiquement acceptable et un antagoniste des récepteurs glutamatergiques NMDA.

## Description

La présente invention concerne une nouvelle association entre le 4-{3-[*cis-*hexahydrocyclopenta[*c*]pyrrol-2(1*H*)-yl]propoxy}benzamide de formule (I): 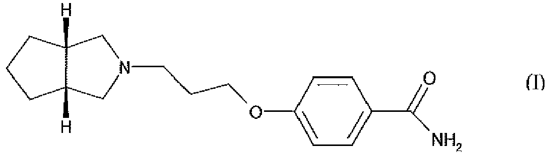 ou un de ses sels d'addition à un acide ou à une base pharmaceutiquement acceptable, et un antagoniste des récepteurs glutamatergiques NMDA (N-methyl-D-aspartate) pour l'obtention de compositions pharmaceutiques utiles dans le traitement des troubles cognitifs associés au vieillissement cérébral et aux maladies neurodégénératives.

Le 4-{3-[*cis*-hexahydrocyclopenta[*c*]pyrrol-2(1*H*)-yl]propoxy}benzamide présente la particularité d'interagir avec les systèmes histaminergiques centraux *in vivo.* Ces propriétés lui confèrent une activité dans le système nerveux central et plus particulièrement dans le traitement des déficits cognitifs associés au vieillissement cérébral et aux maladies neurodégénératives.

Le 4-{3-[*cis*-hexahydrocyclopenta[*c*]pyrrol-2(1*H*)-yl]propoxy}benzamide, sa préparation et son utilisation en thérapeutique ont été décrits dans la demande de brevet WO2005/089747.

La demanderesse a présentement découvert que le 4-{3-[*cis*-hexahydrocyclopenta[*c*]pyrrol -2(1*H*)-yl]propoxy}benzamide de formule (I), ou ses sels d'addition à un acide ou à une base pharmaceutiquement acceptable, utilisé en association avec un antagoniste des récepteurs glutamatergiques NMDA, possédait des propriétés intéressantes pour le traitement des troubles cognitifs associés au vieillissement cérébral et aux maladies neurodégénératives.

Les maladies neurodégénératives liées au vieillissement cérébral telles que la maladie d'Alzheimer sont caractérisées par des troubles de la mémoire et des dysfonctionnements cognitifs. Les troubles cognitifs sont généralement associés à une diminution de la capacité des neurones à synthétiser et à libérer certains neurotransmetteurs. De plus, on observe une perte progressive de la plasticité synaptique et des processus neuronaux, cette perte neuronale étant accélérée dans certaines régions spécifiques du cerveau. Parmi les divers neurotransmetteurs, l'histamine et l'acétylcholine centrales jouent un rôle crucial dans le contrôle des fonctions cognitives (Witkin and Nelson, Pharmacol. & Therap., 2004, 103, 1-20) et il a été montré que leurs taux diminuaient fortement dans le cerveau des patients atteints par la maladie d'Alzheimer par rapport à ceux observés chez des personnes âgées saines (Panula et al, Neuroscience, 1998, 82(4), 993-997).
Les récepteurs histaminergiques de type H₃, particulièrement abondants dans le système nerveux central, sont des modulateurs principalement présynaptiques de la transmission nerveuse et sont présents dans divers circuits neuronaux en rapport avec la cognition (Blandina et al, Learn Mem., 2004, 11(1): 1-8). Ils agissent en régulant de manière négative la libération de neurotransmetteurs tels que l'histamine, l'acétylcholine, la sérotonine, la noradrénaline et la dopamine. Etant donné que les neurones histaminergiques semblent être largement épargnés dans la maladie d'Alzheimer, les composés antagonistes ou agonistes inverses des récepteurs H₃ pourraient ouvrir la voie à de nouveaux traitements des troubles cognitifs liés au vieillissement cérébral.
A l'inverse, on observe une dégénérescence progressive des neurones cholinergiques au cours de la maladie d'Alzheimer et un dysfonctionnement de la neurotransmission glutamatergique. Cibler le système glutamatergique, spécialement les récepteurs NMDA, offre une approche alternative de traitement par rapport aux médicaments ciblant uniquement le système cholinergique (i.e, les inhibiteurs d'acétylcholinestérases). La mémantine est un antagoniste non compétitif du récepteur NMDA, des récepteurs nicotiniques et du récepteur sérotoninergique 5HT₃, et présente également une composante dopaminergique (Lipton, Nat Rev Drug Discov., 2006, 5(2): 160-70; Aracava et al.,J Pharmacol Exp Ther., 2005, 312(3): 1195-205; Rammes et al., Neurosci Lett., 2001, 306: 81-84). La mémantine est couramment utilisée dans le traitement symptomatique des formes modérées à sévères de la maladie d'Alzheimer. En effet, il a été montré que la mémantine, tout comme les antagonistes /agonistes inverses des récepteurs H₃, permettait d'améliorer les performances cognitives dans différents modèles animaux de mémoire épisodique et de mémoire de travail (Yuede et al, Behav. Pharmacol., 2007, 18(5-6): 347-363). L'amélioration des fonctions cognitives peut donc reposer sur plusieurs types de stratégie, ciblant notamment soit l'histamine, soit le système glutamatergique.

De façon surprenante, la présente invention a montré que les effets des antagonistes des récepteurs glutamatergiques NMDA sont potentialisés par ceux du 4*-*{3*-*[*cis-*hexahydrocyclopenta[*c*]pyrrol-2(1*H*)-yl]propoxy}benzamide ou de ses sels d'addition à un acide ou à une base pharmaceutiquement acceptable. Ainsi, la co-administration de ces composés pourrait permettre d'améliorer les performances cognitives des patients par rapport à la simple administration d'un antagoniste des récepteurs glutamatergiques NMDA sans toutefois augmenter les effets délétères associés au traitement par les antagonistes des récepteurs glutamatergiques NMDA (en particulier, la somnolence, les céphalées, les sensations vertigineuses, l'hypertension, la dyspnée ou la constipation). Autrement dit, des traitements associant le 4-{3-[*cis*-hexahydrocyclopenta[*c*]pyrrol-2(1*H*)-yl]propoxy}benzamide à des doses thérapeutiques d'antagonistes des récepteurs NMDA inférieures à celles classiquement utilisées en mono-administration deviennent donc envisageables, avec des performances cognitives équivalentes voire supérieures et des effets délétères moindres.

Cet effet, non prévisible, permet d'envisager l'utilisation d'une association entre le 4-{3-[*cis*-hexahydrocyclopeta[*c*]pyrrol-2(1*H*)-yl]propoxy}benzamide, ou un de ses sels d'addition, et un antagoniste des récepteurs NMDA dans le traitement des troubles cognitifs associés au vieillissement cérébral et aux maladies neurodégénératives. Les troubles cognitifs associés à la maladie d'Alzheimer sont particulièrement visés.

Préférentiellement, le 4-{3-[*cis-*hexahydrocyclopenta[*c*]pyrrol-2(1*H*)-yl]propoxy} benzamide est utilisé sous la forme d'un oxalate ou d'un chlorhydrate dans le cadre de l'invention.

Parmi les antagonistes des récepteurs NMDA selon l'invention, on peut citer la mémantine, la L-4-chlorokynurénine, la 1-(2,2-diphényltetrahydrofuran-3-yl)-*N*,*N-*diméthylméthanamine (ANAVEX 2-73) et la (5*R*,9*R*,11*E*)-5-amino-11-éthylidene-7-méthyl-5,6,9,10-tétrahydro-5,9-méthanocycloocta[*b*]pyridin-2(1*H*)-one (huperzine-A). La mémantine est particulièrement préférée. La mémantine est préférentiellement utilisée sous la forme d'un chlorhydrate.

L'invention concerne donc l'utilisation de l'association entre le 4-{3-[*cis-*hexahydrocyclopenta[*c*]pyrrol-2(1*H*)-yl]propoxy}benzamide ou ses sels d'addition à un acide ou à une base pharmaceutiquement acceptable, et un antagoniste des récepteurs NMDA, pour l'obtention de compositions pharmaceutiques destinées au traitement des troubles cognitifs associés au vieillissement cérébral et aux maladies neurodégénératives.

Plus particulièrement, l'association du 4-{3-[*cis*-hexahydrocyclopenta[*c*]pyrrol-2(1*H*)-yl]propoxy}benzamide et de la mémantine est utilisée dans le traitement des troubles cognitifs associés à la maladie d'Alzheimer.

L'invention concerne également les compositions pharmaceutiques contenant l'association entre le 4-{3-[*cis*-hexahydrocyclopenta[*c*]pyrrol-2(1*H*)-yl]propoxy}benzamide ou ses sels d'addition à un acide ou à une base pharmaceutiquement acceptable, et un antagoniste des récepteurs NMDA en combinaison avec un ou plusieurs excipients pharmaceutiquement acceptables.

Dans les compositions pharmaceutiques selon l'invention, la fraction massique en principes actifs (masse des principes actifs sur la masse totale de la composition) est comprise entre 5 et 50%.

Parmi les compositions pharmaceutiques selon l'invention, on retiendra plus particulièrement celles qui conviennent pour l'administration par voie orale, parentérale et notamment intraveineuse, per ou transcutanée, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades, gels dermiques, etc...

Outre le 4-{3-[*cis*-hexahydrocyclopenta[*c*]pyrrol-2(1*H*)-yl]propoxy}benzamide et le composé antagoniste des récepteurs NMDA, les compositions pharmaceutiques selon l'invention contiennent un ou plusieurs excipients ou véhicules choisis parmi des diluants, des lubrifiants, des liants, des agents de désintégration, les stabilisants, les conservateurs, des absorbants, des colorants, des édulcorants, les aromatisants etc...

### A titre d'exemple et de manière non limitative, on peut citer:

◆ *pour les diluants:* le lactose, le dextrose, le sucrose, le mannitol, le sorbitol, la cellulose, la glycérine,
◆ *pour les lubrifiants:* la silice, le talc, l'acide stéarique et ses sels de magnésium et de calcium, le polyéthylène glycol,
◆ *pour les liants:* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants:* l'agar, l'acide alginique et son sel de sodium, les mélanges effervescents.

Les composés de l'association peuvent être administrés de manière simultanée ou séquentielle. La voie d'administration préférée est la voie orale et les compositions pharmaceutiques correspondantes peuvent permettre la libération instantanée ou différée des principes actifs. Par ailleurs, les composés de l'association peuvent être administrés sous la forme de deux compositions pharmaceutiques distinctes, contenant chacune l'un des principes actifs, ou bien sous la forme d'une seule composition pharmaceutique, dans laquelle les principes actifs sont mélangés.

Les compositions pharmaceutiques préférées sont les comprimés.

La posologie utile varie selon le sexe, l'âge et le poids du patient, la voie d'administration, la nature de l'affection et des traitements éventuellement associés et s'échelonne entre 0,5 mg et 100 mg de 4-{3-[*cis*-hexahydrocyclopenta[*c*]pyrrol-2(1*H*)-yl]propoxy}benzamide par 24 heures, et plus préférentiellement 2 mg, 5 mg ou 20 mg (exprimé en équivalent base) par jour. La dose de l'antagoniste des récepteurs NMDA sera égale ou inférieure à celle utilisée lorsqu'il est administré seul. Dans le cas de la mémantine, la posologie est comprise entre 1 mg et 20 mg par jour, les doses quotidiennes préférées étant de 10 et 20 mg pour le chlorhydrate de mémantine.

Dans les modes de réalisation préférés de l'invention, l'association entre le 4-{3*-*[*cis-*hexahydrocyclopenta[*c*]pyrrol-2(1*H*)-yl]propoxy}benzamide (composé S) et la mémantine est administrée aux doses suivantes :

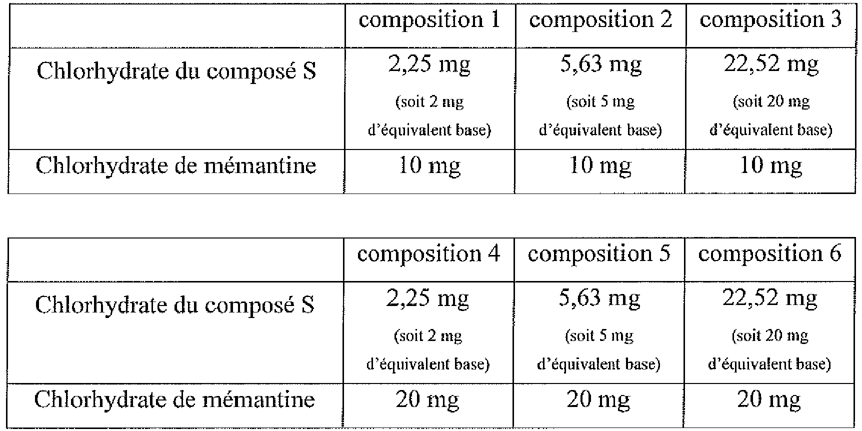

### Composition pharmaceutique:

Formule de préparation pour 1000 comprimés dosés à 5,63 mg de chlorhydrate de 4-{3-[*cis*-hexahydrocyclopenta[*c*]pyrrol-2(1*H*)-yl]propoxy}benzamide (correspondant à 5 mg d'équivalent base) et 10 mg de chlorhydrate de mémantine:

| | |
|---|---|
| Chlorhydrate de 4-{3-[*cis*-hexahydrocyclopenta[*c*]pyrrol-2(1*H*)-yl]propoxy}benzamide | 5,63 g |
| Chlorhydrate de mémantine | 10 g |
| Amidon de mais | 20 g |
| Maltodextrine | 7,5 g |
| Silice colloïdale | 0,2 g |
| Glycolate d'amidon de sodium | 3 g |
| Stéarate de magnésium | 1 g |
| Lactose | 55 g |

### EXEMPLE A:

### Expérience dans un modèle de mémoire épisodique, le test de discrimination contextuelle en série:

Les effets du 4-{3-[*cis*-hexahydrocyclopenta[*c*]pyrrol-2(1*H*)-yl]propoxy}benzamide et de la mémantine (tous deux utilisés sous la forme d'un chlorhydrate), administrés seuls ou en combinaison, ont été étudiés en utilisant un test de discrimination contextuelle chez la souris C57B16 d'âge moyen (14-15 mois) (n=12 par groupe) (Célérier et al, Learn Mem., 2004, 11(2): 196-204; Tronche et al., Behav. Brain Res., 2010, 215(2): 255-60). Dans ce modèle, les souris d'âge moyen présentent par rapport aux souris jeunes un dysfonctionnement spécifique de la mémoire épisodique contextuelle, sans déficit de mémoire spatiale. Ce modèle est pertinent pour évaluer les effets des produits dans la maladie d'Alzheimer car les patients atteints de cette démence présentent également, et ce de manière très précoce, des troubles de mémoire épisodique contextuelle (Gold and Budson, Expert Rev Neurother., 2008, 8(12): 1879-1891).

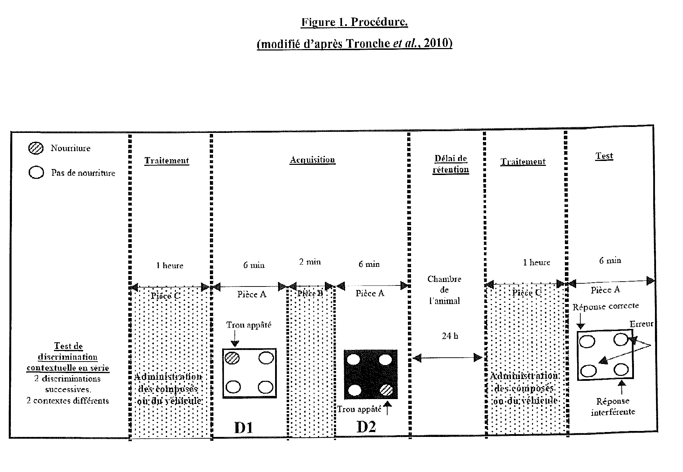

Les souris, placées dans une boîte à bords élevés, apprennent deux types de discriminations spatiales consécutives (D1: plancher blanc puis D2: plancher noir) sur un plancher à quatre trous, dans lequel seul un des trous est appâté, et ce de manière opposée entre D1 et D2. La couleur du plancher (blanc ou noir) constitue le contexte interne spécifique à chaque discrimination. 24h après l'étape d'apprentissage, les souris sont replacées sur le plancher contextuel blanc et sont mesurés:
- le pourcentage de réponses correctes (i.e. % d'inclinaisons de la tête dans le trou ayant été appâté pendant l'exercice d'acquisition sur le plancher blanc),
- le pourcentage de réponses interférentes (i.e. % d'inclinaisons de la tête dans le trou ayant été appâté pendant l'exercice d'acquisition sur le plancher noir, le dernier contexte présenté aux souris),
- et le pourcentage d'erreurs (i.e. % d'inclinaisons de la tête dans les deux trous n'ayant pas été appâtés pendant l'acquisition, ni sur le plancher blanc, ni sur le plancher noir) (voir Figure 1).

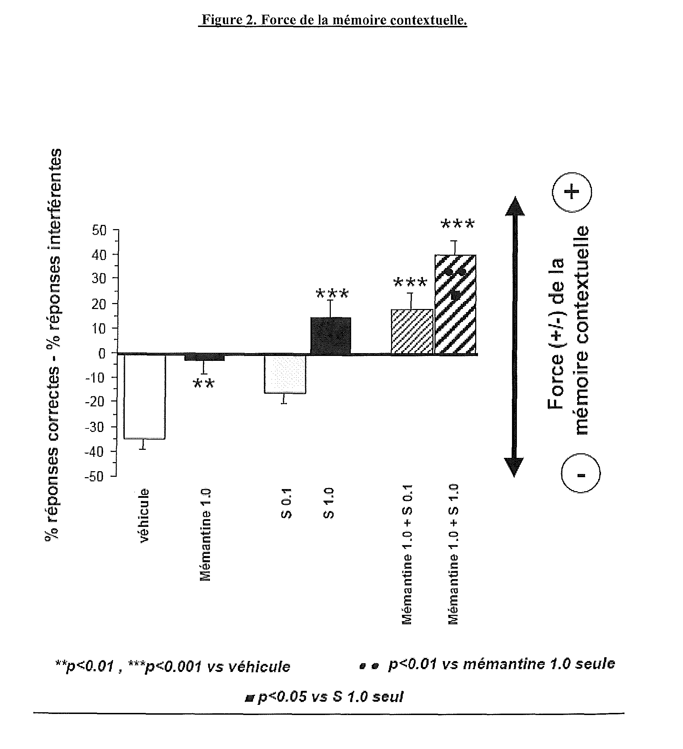

La force de la mémoire contextuelle est définie comme étant la différence entre le pourcentage de réponses correctes et le pourcentage de réponses interférentes.
Dans ce modèle, il a été montré que les souris d'âge moyen présentent un déficit de mémoire contextuelle par rapport aux souris jeunes, dû au fait que le denier contexte où elles ont appris la localisation du trou appâté (i.e. plancher noir) interfère de façon importante avec la mémoire du trou appâté dans le premier contexte présenté lors de l'acquisition (i.e. plancher blanc). De ce fait, les souris âgées ont des valeurs de force de la mémoire contextuelle négatives puisque le pourcentage de réponses interférentes est supérieur au pourcentage de réponses correctes. A l'inverse, les souris jeunes présentent une force de la mémoire contextuelle positive (Tronche et al., Behav. Brain Res., 2010, 215(2): 255-60).
Les résultats de cette étude confirment le déficit de mémoire contextuelle des souris d'âge moyen: les souris véhicules de cette expérience montrent en effet une force de la mémoire contextuelle négative de -34%. Après un traitement chronique de 9 jours de chlorhydrate de 4-{3-[*cis*-hexahydrocyclopenta[*c*]pyrrol-2(1*H*)yl]propoxy}benzamide (0,1 mg/kg de base per os, composé appelé S sur la Figure 2), on n'observe aucune augmentation significative de la force de la mémoire contextuelle par rapport au véhicule (-15% versus - 34%), le % de réponses interférentes restant supérieur au % de réponses correctes. Par ailleurs, la force de la mémoire contextuelle augmente légèrement par rapport au véhicule après un traitement chronique de 9 jours de chlorhydrate de mémantine à la dose de 1 mg/kg de base per os (-2% versus -34%), mais reste négative (% réponses interférentes > % réponses correctes) faisant donc considérer la dose de 1 mg/kg de chlorhydrate de mémantine comme une dose subactive. En revanche, l'administration de l'association du 4-{3-[*cis*-hexahydracyclopenta[*c*]pyrrol-2(1*H*)-yl]propoxy}benzamide (0,1 mg/kg de base per os) avec la mémantine (dose subactive de 1 mg/kg de base per os) conduit à une augmentation importante et significative de la force de la mémoire contextuelle par rapport à la valeur obtenue avec le véhicule seul, la force de la mémoire contextuelle devenant alors positive (% réponses correctes > % réponses interférentes). Ces résultats montrent une potentialisation nette des effets de la mémantine à dose subactive en présence d'une dose inactive de 4-{3-[*cis*-hexahydrocyclopenta[*c*]pyrrol-2(1*H*)yl]propoxy}benzamide. Cette potentialisation est également confirmée lorsque la mémantine (1 mg/kg de base per os) est associée à une dose active de 4-{3-[*cis*-hexahydrocyclopenta[*c*]pyrrol-2(1*H*)-yl]propoxy}benzamide (1 mg/kg de base per os): on observe alors un accroissement important de la performance mnésique des souris traitées par rapport à la mémantine seule, et ce de manière statistiquement significative (force de la mémoire contextuelle de +40%: l'association donne une réponse très positive versus une réponse négative de -2% pour la mémantine seule). Cet accroissement de la performance mnésique des souris traitées par l'association est également confirmé par rapport au 4-{3-[*cis-*hexahydrocyclopenta[*c*]pyrrol-2(1*H*)-yl]propoxy}benzamide seul (1 mg/kg de base per os) (force de la mémoire contextuelle de +40% pour l'association versus +14% pour le 4-{3-[*cis*-hexahydrocyclopenta[*c*]pyrrol-2(1*H*)-yl]propoxy}benzamide), et ceci aussi de manière statistiquement significative. L'augmentation de la force de la mémoire contextuelle observée pour les deux associations ne peut pas s'expliquer par une simple additivité des effets des composés administrés seuls, et montre une synergie d'activité des deux composés lorsqu'ils sont co-administrés.

Les résultats mettent clairement en évidence que l'administration de ces deux composés en association permet d'obtenir un effet synergique important tout à fait inattendu. Par ailleurs, les analyses pharmacocinétiques ont montré qu'il n'y avait aucune interaction de type pharmacocinétique entre les deux traitements qui pourrait justifier ou interférer avec l'effet synergique décrit ci-dessus.

En conclusion, les résultats présentés ci-dessus mettent en évidence une synergie d'activité entre le 4-{3-[*cis*-hexahydrocyclopenta[*c*]pyrrol-2(1*H*)-yl]propoxy}benzamide et la mémantine en terme de performances cognitives, et ce sans interaction pharmacocinétique.

## Revendications

1. Association entre le 4-{3-[*cis*-hexahydrocyclopenta[*c*]pyrrol-2(1*H*)-yl]propoxy} benzamide de formule (I): 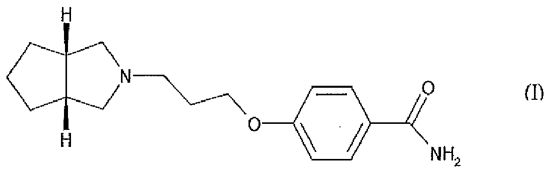 ou un de ses sels d'addition à un acide ou à une base pharmaceutiquement acceptable, et un antagoniste des récepteurs glutamatergiques NMDA.

2. Association selon la revendication 1 **caractérisée en ce que** le 4-{3-[*cis-*hexahydrocyctopenta[*c*]pyrrol-2(1*H*)-yl]propoxy}benzamide est utilisé sous la forme d'un oxalate ou d'un chlorhydrate.

3. Association selon la revendication 1 ou 2 dans laquelle l'antagoniste des récepteurs glutamatergiques NMDA est la mémantine.

4. Association selon l'une des revendications 1 à 3 dans laquelle la mémantine est utilisée sous la forme d'un chlorhydrate.

5. Association selon l'une des revendications 1 à 4 pour son utilisation en tant que médicament dans laquelle : (i) le 4-{3-[*cis*-hexahydrocyclopenta[*c*]pyrrol-2(1*H*)-yl]propoxy}benzamide est administré à la dose journalière de 2 mg, 5 mg ou 20 mg (exprimée en équivalent base) sous la forme d'un chlorhydrate , et (ii) l'antagoniste des récepteurs glutamatergiques NMDA est le chlorhydrate de mémantine administré à la dose journalière de 10 mg ou 20 mg.

6. Composition pharmaceutique contenant comme principe actif le 4-{3-[*cis-*hexahydrocyclopenta[*c*]pyrrol-2(1*H*)-yl]propoxy}benzamide, ou un de ses sels d'addition à un acide ou à une base pharmaceutiquement acceptable, en association avec un antagoniste des récepteurs glutamatergiques NMDA selon l'une des revendications 1 à 4 en combinaison avec un ou plusieurs excipients pharmaceutiquement acceptables.

7. Composition pharmaceutique selon la revendication 6 pour son utilisation dans le traitement des troubles cognitifs associés au vieillissement cérébral et aux maladies neurodégénératives.

8. Composition pharmaceutique pour son utilisation selon la revendication 7 dans le traitement des troubles cognitifs associés à la maladie d'Alzheimer.

9. Composition pharmaceutique selon la revendication 6 **caractérisée en ce qu'**elle comprend 2 mg, 5 mg ou 20 mg (exprimée en équivalent base) de 4-{3-[*cis-*hexahydrocyclopenta[*c*]pyrrol-2(1*H*)-yl]propoxy}benzamide sous la forme d'un chlorhydrate, et 10 mg ou 20 mg de chlorhydrate de mémantine en tant qu'antagoniste des récepteurs glutamatergiques NMDA.

10. Utilisation d'une association selon l'une des revendications 1 à 5 pour la fabrication d'un médicament destiné au traitement des troubles cognitifs associés au vieillissement cérébral et aux maladies neurodégénératives.

11. Utilisation d'une association selon l'une des revendications 1 à 5 pour la fabrication d'un médicament destiné au traitement des troubles cognitifs associés à la maladie d'Alzheimer.

12. Association selon l'une des revendications 1 à 5 entre le 4-{3-[*cis-*hexahydrocyclopenta[*c*]pyrrol-2(1*H*)-yl]propoxy}benzamide ou un de ses sels d'addition à un acide ou à une base pharmaceutiquement acceptable, et la mémantine, pour son utilisation dans le traitement des troubles cognitifs associés à la maladie d'Alzheimer.

13. Utilisation d'une association selon l'une des revendications 1 à 5 entre le 4-{3-[*cis-*hexahydrocyclopenta[*c*]pyrrol-2(1*H*)-yl]propoxy}benzamide ou un de ses sels d'addition à un acide ou à une base pharmaceutiquement acceptable, et la mémantine pour la fabrication d'un médicament destiné au traitement des troubles cognitifs associés à la maladie d'Alzheimer.
